# EUROPEAN PATENT SPECIFICATION

(11) **EP 1 601 352 B1**
(45) Date of publication and mention of the grant of the patent: **23.01.2008**
(21) Application number: 04720007.6
(22) Date of filing: 12.03.2004
(51) Int. Cl.: A61K 31/192, A61K 31/216, A61K 31/223, A61P 3/04

(54) **COMBINED USE OF A FIBRATE AND ORLISTAT FOR THE TREATMENT OF OBESITY**
KOMBINIERTE VERWENDUNG VON EINEM FIBRAT UND VON ORLISTAT ZUR BEHANDLUNG VON OBESITAS
UTILISATION COMBINEE D'UN FIBRATE ET DE L'ORLISTAT POUR LE TRAITEMENT DE L'OBESITE

(30) Priority: 13.03.2003 EP 03290625
(43) Date of publication of application: 07.12.2005
(73) Proprietor: Fournier Laboratories Ireland Limited, Carrigtwohill, Co. Cork (IE)
(72) Inventor: EDGAR, Alan, F-21490 Saint Julien (FR)
(74) Representative: Hart Davis, Jason
(86) International application number: PCT/EP2004/004010
(87) International publication number: WO 2004/080450

(56) References cited:
- WO-A-01/27128
- WO-A-01/60807
- US-A- 4 598 089
- MANCINI^A^ ^B F P ET AL: "Fenofibrate prevents and reduces body weight gain and adiposity in diet-induced obese rats" FEBS LETTERS, ELSEVIER SCIENCE PUBLISHERS, AMSTERDAM, NL, vol. 491, no. 1-2, 23 February 2001 (2001-02-23), pages 154-158, XP004257293 ISSN: 0014-5793
- TOLENTINO MARSHA C ET AL: "Combination of gemfibrozil and orlistat for treatment of combined hyperlipidemia with predominant hypertriglyceridemia." ENDOCRINE PRACTICE: OFFICIAL JOURNAL OF THE AMERICAN COLLEGE OF ENDOCRINOLOGY AND THE AMERICAN ASSOCIATION OF CLINICAL ENDOCRINOLOGISTS. UNITED STATES 2002 MAY-JUN, vol. 8, no. 3, May 2002 (2002-05), pages 208-212, XP009013738 ISSN: 1530-891X
- COMPAGNON P ET AL: "Effect of orlistat on the pharmacokinetics of repeated doses of sustained-release bezafibrate in healthy volunteers." FUNDAMENTAL & CLINICAL PHARMACOLOGY, vol. 14, no. 3, May 2000 (2000-05), page 246, XP009013728 4th Meeting of the French Pharmacological Society;Rouen, France; April 10-12, 2000 ISSN: 0767-3981

## Description

The present invention relates to the use of a fibrate and orlistat in the manufacture of a medicament to treat patients suffering from obesity.

Tetrahydrolipstatin ("THL") is an inhibitor of pancreatic lipase and is known by the generic name orlistat. The use of THL as a medicament, particularly as an anti-obesity agent, and pharmaceutical compositions containing THL as an active agent are described in U.S. Patent No. 4,598,039. A process for the preparation of orlistat is described in U.S. Patent No. 4,983,746. A pharmaceutical composition comprising orlistat and sibutramine is described in WO 99/33450.

Fibrates, which are PPARα activators, have been reported to lower plasma triglycerides and cholesterol levels and to be beneficial in the prevention of ischemic heart disease in individuals with elevated levels of LDL cholesterol. They can also decrease to some extent elevated fibrinogen and PAI-1 levels. Fibrate compounds, e.g., gemfibrozil, fenofibrate, bezafibrate, and ciprofibrate, elevate the level of plasma HDL cholesterol. A body mass reducing effect is known for the monotherapies with fenofibrate as well as with orlistat (Mancini F P et al. FEBS Letters, vol. 491, no. 1-2, 23 February 2001 and US-A-4 598 089).
Tolentino Marsha C et al. (Endocrine practice, vol. 8, no. 3, May 2002, pages 208-212) discloses the combined use of gemfibrozil and orlistat for the treatment of hyperlipidemia. Compagnon P et al. (Fundamental & Clinical Pharmacology, vol. 14, no. 3, May 2000, page 246) relates to the influence of orlistat on the pharmacokinetics of bezafibrate.

It has now surprisingly been found that co-administration of a fibrate and orlistat results in beneficial effects in obese or overweight subjects.

Accordingly, the present invention relates to the use of a fibrate, orlistat and a pharmaceutically acceptable carrier in the manufacture of a medicament for the treatment of obesity, comprising co-administering an effective dose of a fibrate and orlistat. The fibrate used in this method is selected from the group consisting of gemfibrozil, fenofibrate, bezafibrate, clofibrate and ciprofibrate.

In another embodiment of the invention the use comprises co-administering an effective dose of a fibrate and orlistat, where the effective dose of the fibrate is in the range of about 10 to about 3000 mg per day.

In another embodiment, the effective dose of orlistat is in the range of about 50 to about 1440 mg per day.
In another embodiment, the fibrate and orlistat are administered simultaneously, comprising co-administering an effective dose of a fibrate and orlistat.

In another embodiment of the use for the manufacture of a medicament for the treatment of obesity, the fibrate and orlistat are administered sequentially.

In another embodiment, the invention includes the use for the manufacture of a medicament for the treatment of obesity in a patient already treated with orlistat, which comprises administering to the patient an effective dose of a fibrate. As mentioned above, the fibrate and orlistat are administered simultaneously or sequentially.

As demonstrated in the present specification, the use of a fibrate and orlistat, has led to unexpectedly favourable results in that body weight control is more efficient when orlistat is co-administered with a fibrate.

Furthermore, the applicant has shown that the association of both compounds allows a lower dose of orlistat to be used while maintaining the same therapeutic effects.

The invention also enables the reduction of side effects, as a lower dose of orlistat is used.

As used in this application, "co-administration" means the administration of two or more compounds to the same patient, within a time period of up to about three to about four hours. For example, co-administration encompasses (1) simultaneous administration of a first and second compound; (2) administration of a first compound, followed by administration of a second compound about 2 hours after administration of the first compound; and (3) administration of a first compound, followed by administration of a second compound about 4 hours after administration of the first compound. As described herein, the present invention encompasses co-administration of a fibrate and orlistat to a patient.

In the present invention, the fibrates are defined as PPARα agonists (peroxisome proliferator activated receptor alpha agonists), including the fibric acid derivatives (e.g. fenofibric acid or clofibric acid) and pharmaceutically acceptable salts and esters of such fibric acid derivatives. Fibric acid derivatives lower the levels of triglyceride-rich lipoproteins, such as VLDL, raise HDL levels, and have variable effects on LDL levels. The effects on VLDL levels appear to result primarily from an increase in lipoprotein lipase activity, especially in muscle. This leads to enhanced hydrolysis of VLDL triglyceride content and enhanced VLDL catabolism. Fibric acid agents also may alter the composition of the VLDL, for example, by decreasing hepatic production of apoC-III, an inhibitor of lipoprotein lipase activity. These compounds are also reported to decrease hepatic VLDL triglyceride synthesis, possibly by inhibiting fatty acid synthesis and by promoting fatty acid oxidation. The fibrate compound is selected from gemfibrozil, fenofibrate, bezafibrate, clofibrate, ciprofibrate.

Fenofibrate is commercially available as Tricor® capsules. Each capsule contains 67 mg of micronized fenofibrate.

Fenofibric acid, the active metabolite of fenofibrate, lowers plasma triglycerides apparently by inhibiting triglyceride synthesis, resulting in a reduction of VLDL released into the bloodstream, and also by stimulating the catabolism of triglyceride rich lipoproteins (i.e. VLDL).

Clofibrate is commercially available as Atromid-S® capsules. Each capsule contains 500 mg of clofibrate. Clofibrate lowers elevated serum lipids by reducing the very low-density lipoprotein fraction rich in triglycerides. Serum cholesterol may be decreased. It may inhibit the hepatic release of lipoproteins (particularly VLDL) and potentiate the action of lipoprotein lipase. The recommended daily dose of clofibrate is 2 g, administered in divided doses.

Gemfibrozil is commercially available as Lopid® tablets. Each tablet contains 600 mg of gemfibrozil. Gemfibrozil is a lipid regulating agent that decreases serum triglycerides and very low density lipoprotein cholesterol, and increases high density lipoprotein cholesterol. The recommended daily dose of gemfibrozil is 1200 mg, administered in two divided doses.

The fibrates include PPARα agonists; the PPARα agonists may be identified according to an assay described in US Patent 6,008,239.

According to the present invention, the preferred fibrate is fenofibrate.

Orlistat is commercially available as Xenical® and is indicated in conjunction with a mildly hypocaloric diet for the treatment of obese patients with a body mass index (BMI) greater than or equal to 30 kg/m², or overweight patients (BMI ≥ 28 kg/m²) with associated risk factors.

Chemically, orlistat is [2S-[2α(R*),3β]]-N-formyl-L-leucine 1-[(3-hexyl-4-oxo-2-oxetanyl)methyl]dodecyl ester. It is also known as N-formyl-L-leucine ester with (3S,4S)-3-hexyl-4-[(2S)-2-hydroxy-tridecyl]-2-oxetanone, or (-)-tetrahydrolipstatin.

According to the present invention, a preparation is defined as the formulation of the active compound(s) with encapsulating material as a carrier providing a capsule in which the active component with or without other carriers, is surrounded by a carrier, which is thus in association with it. This includes tablets, powders, capsules, pills, cachets, and lozenges which can be used as solid dose forms suitable for oral administration.

An effective dose is defined in the present invention as the amount of a compound that prevents or ameliorates adverse conditions or symptoms of disease(s) or disorder(s) being treated. With respect to orlistat, the effective dose is in the range of about 50 to about 1440 mg/day given in one or more doses, preferably three times daily, preferably in the range of about 120 to about 720 mg/day and more preferably in the range of about 120 to about 360 mg/day. Orlistat is preferably administered orally. With respect to the fibrate, the effective dose is in the range of about 10 to about 3000 mg/day given in one or more doses, preferably in the range of about 50 to about 1200 mg/day, and more preferably in the range of about 50 to about 300 mg/day. The skilled artisan will understand and appreciate that the effective dose of a given fibrate will vary with the potency of the fibrate.

The present invention relates to the unexpected discovery that co-administration of a fibrate and orlistat exerts beneficial effects in overweight or obese subjects, i.e. subjects having a BMI ≥ 28 kg/m².

The fibrate is selected from the group consisting of gemfibrozil, fenofibrate, bezafibrate, clofibrate and ciprofibrate; fenofibrate being the preferred fibrate.

The effective dose of the fibrate is in the range of about 10 to about 3000 mg per day and the effective dose of orlistat is in the range of about 50 to about 1440 mg per day.

According to the invention, the fibrate and orlistat can be administered simultaneously, or sequentially. In a preferred embodiment of the invention, the fibrate and orlistat are administered simultaneously, more preferably in one formulation containing the fibrate and orlistat.

Pharmaceutical formulations of the fibrate and/or orlistat molecules can be prepared according to known methods. The preferred route of administering the fibrate and orlistat is mucosal administration, most preferably oral administration.

For preparing pharmaceutical compositions containing a fibrate and/or orlistat, pharmaceutically acceptable carriers can be either solid or liquid. Solid form preparations include powders, tablets, pills, capsules, cachets, suppositories, and dispersible granules. A solid carrier can be one or more substances which may also act as diluents, flavoring agents, binders, preservatives, tablet disintegrating agents, or an encapsulating material.

In powders, the carrier is a finely divided solid which is in a mixture with the finely divided active component(s). In tablets, the active component(s) is (are) mixed with the carrier having the necessary binding properties in suitable proportions and compacted in the shape and size desired. The powders and tablets preferably contain from five or ten to about seventy percent of the active component(s). Suitable carriers are magnesium carbonate, magnesium stearate, talc, sugar, pectin, dextrin, starch, gelatin, tragacanth, methylcellulose, sodium carboxymethylcellulose, a low melting wax, cocoa butter, and the like.

Liquid form preparations include solutions, suspensions, and emulsions, for example, water or water propylene glycol solutions. For parenteral injection liquid preparations can be formulated in solution e.g. in aqueous polyethylene glycol solution.

Aqueous solutions suitable for oral use can be prepared by dissolving the active component in water and adding suitable colorants, flavors, stabilizing and thickening agents as desired. Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

The pharmaceutical preparation is preferably in unit dosage form. In such form the preparation is subdivided into unit doses containing appropriate quantities of the active component(s). The unit dosage form can be a packaged preparation, the package containing discrete quantities of the preparation, such as packeted tablets, capsules, and powders in vials or ampoules. Also, the unit dosage form can be a capsule, tablet, cachet, or lozenge itself, or it can be the appropriate number of any of these in packaged form.

The amount of each compound to be administered will depend on a number of factors including the age of the patient, the severity of the condition and the past medical history of the patient. It is generally envisaged that each unit dose contains (1) from about 10 to about 1000 mg, preferably about 50 to 600 mg, more preferably about 50 to about 200 mg of fibrate, and/or (2) from about 50 to about 720 mg, preferably about 120 to about 360 mg of orlistat. Typical unit doses contain 67 mg, 140 mg, 160 mg, 200 mg, 500 mg or 600 mg of fibrate and/or 120 mg of orlistat.

The invention is further illustrated by the following example, which is not to be construed as limiting, but merely as an illustration of some preferred features of the invention.

### EXAMPLE: Effect of a fibrate and orlistat co-administration on body weight

This study was designed to evaluate the effects of using a combination of fenofibrate and orlistat on body weight.

The data from this study, which are summarized in Table 1, demonstrate that after 6 weeks of treatment (1) there is a significant difference between mice with a high-fat diet, treated with fenofibrate alone or with orlistat alone, and mice with a standard diet (no normalization of the body weight is observed in treated mice), and (2) there is no difference between mice with a high-fat diet, treated with a combination of fenofibrate and orlistat, and mice with a standard diet (the body weight of the treated mice is normalized).

Therefore, better control of the body weight is obtained when orlistat is administered in combination with a fibrate.

### METHOD

### Animals:

Mice weighing approximately 20 g were received from CERJ. They were put into individual cages in a temperature-, humidity- and light- controlled room (21-23°C, 12-12h light-dark cycle). They were fed with either a standard laboratory diet or a high-fat diet, and had free access to water. After acclimatization, they were randomized into groups of 20 animals, based on body weight.
The experimental groups were:
- Group 1: mice fed with high-fat diet
- Group 2: mice fed with high-fat diet, treated with fenofibrate 25 mg/kg p.o., mixed with the diet
- Group 3: mice fed with high-fat diet, treated with orlistat 2.5 mg/kg p.o., mixed with the diet
- Group 4: mice fed with high-fat diet, treated with fenofibrate 25 mg/kg p.o. and orlistat 2.5 mg/kg p.o., mixed with the diet
- Group 5: mice fed with standard diet

*Statistics :* All data are presented as mean ± sem. Results were subjected to Dunnett's t test. A p < 0.05 was considered significant.

**Table 1**

| | **Initial body weight** | **Body weight after 6 weeks** | **Gain** |
|---|---|---|---|
| Group 1 | 19.60 ± 0.18 | 28.93 ± 0.41 | 9.33 ± 0.30* |
| Group 2 | 19.65 ± 0.22 | 27.25 ± 0.47 | 7.60 ± 0.43* |
| Group 3 | 19.53 ± 0.25 | 27.12 ± 0.39 | 7.59 ± 0.28* |
| Group 4 | 19.64 ± 0.28 | 26.46 ± 0.28 | 6.82 ± 0.26 |
| Group 5 | 19.42 ± 0.20 | 25.92 ± 0.42 | 6.50 ± 0.29 |

| | | | |
|---|---|---|---|
| * p< 0.05 relative to Group 5 | | | |

## Claims

1. Use of a fibrate, orlistat and a pharmaceutically acceptable carrier in the manufacture of a medicament for the treatment of obesity, wherein the fibrate is selected from the group consisting of gemfibrozil, fenofibrate, bezafibrate, clofibrate and ciprofibrate.

2. The use according to claim 1, wherein the fibrate is fenofibrate.

3. The use according to claim 1 or claim 2, wherein the fibrate and orlistat are administered simultaneously or sequentially.

4. The use according to one of claims 1 to 3, wherein the effective dose of the fibrate is in the range of about 10 to about 3000 mg per day.

5. The use according to one of claims 1 to 4, wherein the effective dose of orlistat is in the range of about 50 to about 1440 mg per day.

6. A pharmaceutical composition containing a fibrate, orlistat and a pharmaceutically acceptable carrier, wherein the fibrate is selected from the group consisting of gemfibrozil, fenofibrate, bezafibrate, clofibrate and ciprofibrate.

7. The pharmaceutical composition according to claim 6, wherein the fibrate is fenofibrate.

8. The pharmaceutical composition according to claim 6 or claim 7, containing from about 10 to about 1000 mg of fibrate.

9. The pharmaceutical composition according to one of claims 6 to 8, containing from about 50 to about 720 mg of orlistat.

## Patentansprüche

1. Verwendung eines Fibrats, Orlistats und eines pharmazeutisch verträglichen Trägers bei der Herstellung eines Medikamentes zur Behandlung von Fettleibigkeit, wobei das Fibrat gewählt ist aus der Gruppe, die besteht aus Gemfibrozil, Fenofibrat, Bezafibrat, Clofibrat und Ciprofibrat.

2. Verwendung nach Anspruch 1, wobei das Fibrat Fenofibrat ist.

3. Verwendung nach Anspruch 1 oder 2, wobei das Fibrat und Orlistat gleichzeitig oder sequentiell verabreicht werden.

4. Verwendung nach einem Anspruch 1 bis 3, wobei die wirksame Dosis des Fibrats im Bereich von etwa 10 bis etwa 3000 mg pro Tag ist.

5. Verwendung nach einem der Ansprüche 1 bis 4, wobei die effektive Dosis von Orlistat im Bereich von etwa 50 bis etwa 1440 mg pro Tag ist.

6. Pharmazeutische Zusammensetzung, die ein Fibrat, Orlistat und einen pharmazeutisch verträglichen Träger enthält, wobei das Fibrat gewählt ist aus der Gruppe, die besteht aus Gemfibrozil, Fenofibrat, Bezafibrat, Clofibrat und Ciprofibrat.

7. Pharmazeutische Zusammensetzung nach Anspruch 6, wobei das Fibrat Fenofibrat ist.

8. Pharmazeutische Zusammensetzung nach Anspruch 6 oder Anspruch 7, welche von etwa 10 bis etwa 1000 mg Fibrat enthält.

9. Pharmazeutische Zusammensetzung nach einem der Ansprüche 6 bis 8, welche von etwa 50 bis etwa 720 mg Orlistat enthält.

## Revendications

1. Utilisation d'un fibrate, de l'orlistat et d'un support pharmaceutiquement acceptable dans la fabrication d'un médicament pour le traitement de l'obésité, où le fibrate est choisi dans le groupe consistant en gemfibrozil, fénofibrate, bézafibrate, clofibrate et ciprofibrate.

2. Utilisation selon la revendication 1, où le fibrate est le fénofibrate.

3. Utilisation selon la revendication 1 ou la revendication 2, où le fibrate et l'orlistat sont administrés simultanément ou successivement.

4. Utilisation selon l'une des revendications 1 à 3, où la dose efficace de fibrate est dans la plage d'environ 10 à environ 3000 mg par jour.

5. Utilisation selon l'une des revendications 1 à 4, où la dose efficace d'orlistat est dans la plage d'environ 50 à environ 1440 mg par jour.

6. Composition pharmaceutique contenant un fibrate, de l'orlistat et un support pharmaceutiquement acceptable, où le fibrate est choisi dans le groupe consistant en gemfibrozil, fénofibrate, bézafibrate, clofibrate et ciprofibrate.

7. Composition pharmaceutique selon la revendication 6, où le fibrate est le fénofibrate.

8. Composition pharmaceutique selon la revendication 6 ou la revendication 7 contenant d'environ 10 à environ 1000 mg de fibrate.

9. Composition pharmaceutique selon l'une des revendications 6 à 8 contenant d'environ 50 à environ 720 mg d'orlistat.
